# EUROPEAN PATENT SPECIFICATION

(11) **EP 1 896 091 B1**
(45) Date of publication and mention of the grant of the patent: **22.06.2011**
(21) Application number: 06786300.1
(22) Date of filing: 30.06.2006
(51) Int. Cl.: A61L 29/16, A61L 31/16, A61L 31/10, A61L 29/08

(54) **NITRIC OXIDE COATINGS FOR MEDICAL DEVICES**
STICKSTOFFOXIDBESCHICHTUNGEN FÜR MEDIZINISCHE VORRICHTUNGEN
REVETEMENTS D'OXYDE NITRIQUE POUR DISPOSITIFS MEDICAUX

(30) Priority: 30.06.2005 US 695267 P
(43) Date of publication of application: 12.03.2008
(73) Proprietor: Accord Biomaterials, Inc., Ann Arbor, MI 48103 (US); The Regents of the University of Michigan, Ann Arbor, MI 48109 (US)
(72) Inventor: MERZ, Scott I., Ann Arbor, MI 48103 (US); REYNOLDS, Melissa, Ann Arbor, MI 48103 (US); MEYERHOFF, Mark E., Ann Arbor, MI 48103 (US); NANDIVADA, Himabindu, Ann Arbor, MI 48105 (US); HWANG, Sangyeul, Ann Arbor, MI 48105 (US); LAHANN, Joerg, Ann Arbor, MI 48105 (US)
(74) Representative: Gordon, Kirsteen Helen
(86) International application number: PCT/US2006/026101
(87) International publication number: WO 2007/005910

(56) References cited:
- WO-A-01/10344
- WO-A-01/26702
- WO-A-01/85227
- WO-A-2004/012874
- WO-A-2006/037105
- US-A1- 2002 115 559

## Description

### INTRODUCTION

Circulatory diseases are a leading cause of death in the Western world. Although cardiovascular disease is often thought to primarily affect men and the aged, it has been found to be gender-unspecific and heavily affects people in the prime of life. More than half of all cardiovascular disease deaths each year occur among women. Among patients diagnosed with cardiovascular diseases, more than 400,000 undergo cardiovascular intervention every year via placement of coronary artery stents. One prevailing problem following placement of a coronary artery stent is the occurrence of unwanted inflammatory responses. For example, sub-acute and acute thrombosis and hypersensitivity are events that typically occur within the first 30 days following the stenting procedure and can occur with any stent, bare metal or a drug-eluting stent, according to the FDA. In the case of drug eluting stents, such unwanted inflammatory responses may also be due at least in part to organic solvents necessary for coating a stent.

A large number of materials are currently employed to prepare blood-contacting and tissue-implantable medical devices such as vascular grafts, intravascular catheters, coronary artery and vascular stents, insulation materials for electrical leads of pacemakers and defibrillators, extracorporeal bypass circuits, and oxygenators, et. The incompatibility of these materials with human blood and tissue can cause serious complications in patients, and ultimately functional device failure. Implantation of devices into blood vessels causes damage to the endothelial layers and an almost immediate inflammatory response throughout the implant site. For example, in addition to opening the artherosclerotically obstructed artery, placement of a vascular stent may cause endothelial disruption, fracture of internal lamina and dissection of the media of the diseased vessel. Within three to seven days post injury, several processes may occur including adhesion, and the recruitment and activation of neutrophils, monocytes and lymphocytes in an attempt to destroy the foreign body.

the use of NO releasing coatings on stents, shunts and other long-term biomedical implants may be limited by the small reservoir of NO adducts (e.g. diazeniumdiolates) that can be loaded within the coatings of polymeric materials, while simultaneously keeping the coatings non-obtrusive. WO01/85227, WO2004/012874, WO01/26702, WO01/10344 and WO2006/0371 disclose devices that comprise nitric oxide releasing agents. US2002/115559 discloses devices having a nitric oxide generating material. Some devices such as vascular stents may require very thin outer coatings to be functional.

A need exists for the development of an effective and safe biomaterial coating technology that enables immobilization of a very thin layer of a NO generating material, and that may be substantially free of contaminants often present as a result of the coating process.

### SUMMARY OF THE INVENTION

It is an object of the invention, at least in part, to provide nitric oxide generating biomedical coatings.

Provided herein is a medical device substantially free of organic solvent capable of generating a pharmaceutically effective amount of nitric oxide and suitable for insertion into a mammal comprising: a metal layer; a coating with a thickness of about 10 run to about 2000 nm on said metal layer ; where the coating comprises a biocompatible polymer comprising at least one residue covalently bonded to a nitric oxide generating compound. The coating may be about 20 nm to about 200 nm thick.

In some embodiments, disclosed medical devices include a nitric oxide generating compound that is a metal ion binding moiety, such as Nₓ-donor ligand where x is 2, 3, 4, 5, 6, 7, 8 or 9, or a S_{y}-donor ligand where y is 2, 3, 4, 5, 6, 7, 8 or 9. The medical devices may further comprise metal ions, where the metal ions are selected from the ions of one or more of: Cu, Co, Zn, Ca, Mg, Pt, Sn, Se, or Mn.

In some embodiments, the medical device includes a polymer that comprises polymerized [22]paracyclophanes and/or a metal layer that comprises at least one of stainless steel, titanium, copper, gold, nickel, or alloys thereof.

The disclosed medical devices includes such devices a catheter, a stent, a graft, or a pacemaker lead.

Another embodiment provides for a medical device as described herein suitable for implantation to a patient comprising a metal layer; a nitric oxide generating coating with a thickness of 20 nm to 2000 nm on said metal layer; wherein said coating comprises a polymer comprising at least one residue covalently bonded to a nitric oxide generating compound; wherein i said medical device prevents an inflammatory response as compared to the inflammatory response obtained by implanting a medical device with a coating that does not include a nitric oxide generating compound. The disclosed coatings and/or medical devices are free of organic solvents.

Also described herein is a method of preventing an unwanted inflammatory response in a patient comprising: providing the coating on the medical device of disclosed herein with an effective amount of nitric oxide generating compound; and implanting the medical device into a patient in need thereof. In some instances the unwanted inflammatory response is sub-acute thrombosis.

Also described herein is a method for substantially preventing or treating unwanted inflammatory response in a patient in need of an implantable.medical device, comprising:

implanting a medical device into patient thereby providing a pharmaceutically effective amount of nitric oxide to said patient, wherein said medical device comprises a metal layer a coating with a thickness of 20 nm to 2000 nm on said metal layer; wherein the coating comprises a polymer and a nitric oxide generating compound.

A method for preparing a nitric oxide generating coating is described comprising: providing a first moiety comprising a cyclen moiety; providing a second moiety comprising a photo-activatable residue; and irradiating or heating the first moiety and the second moiety.
Another method for preparing a nitric oxide generating coating is described comprising providing a first moiety comprising a cyclen moiety covalently bonded to photo-activatable monomer; providing a second moiety comprising a poly(xylylene); and irradiating or heating the first moiety and the second moiety simultaneously. A further method for preparing a nitric oxide generating coating comprising providing a first moiety comprising a cyclen moiety covalently bonded to a polymer comprising a photo-activatable residue; providing a second moiety comprising a poly(xylylene); and irradiating or heating the first moiety and the second moiety simultaneously is disclosed.

Also described herein is the use of a cyclen bonded to a polymer for use in medical devices.

The present invention provides a number of methods of making the subject compositions. Examples of such methods include those described in the Exemplification below.

### BRIEF DESCRIPTION OF THE FIGURES

Figure 1 pictorially illustrates exemplary synthetic approaches for the fabrication of Cu(II)-ligand coatings.

Figure 2 depicts a synthesis of isolated crosslinked polymethacryate polymers containing pendant Cu(II)-cyclen complexes. Boc = t-butoxycarbonyl protecting group.

Figure 3 depicts a route to attach crosslinked methacrylate polymers containing pendant Cu(II)- cyclen complexes to CVD PPX polymer.

Figure 4 depicts an infrared spectrum comparing a (top) CVD coating and (bottom) polymethacrylate/Cu(II)cyclen CVD coating on stainless steel substrates.

Figure 5 depicts a NO generation curve from CVD/polymethacrylate/Cu(II)-cyclen coatings on stainless steel in the presence of 10 µM GSNO and 30 µM GSH. Each peak represents a new injection of GSNO.

Figure 6 depicts an illustration of surface chemistry that can be used to prepare "brush-like" chains of Cu(II) ligands on a surface.

Figure 7 pictorially represents CVD polymerization of various functionalized poly-p-xylenes.

Figure 8 depicts an SEM image of a CVD coated stent after loading and release from a catheter set. The coating was partially damaged by the procedure. The arrow indicates stretch pattern due to stent expansion.

Figure 9 depicts a Cu-cyclen-pHEMA hydrogel with varying copper content.

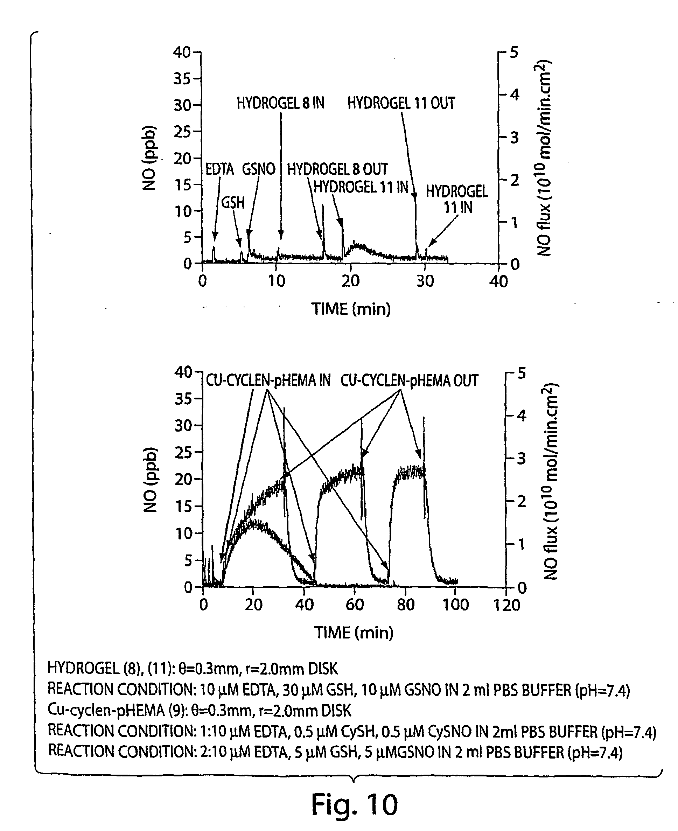

Figure 10 depicts nitric oxide generation from endogenous substrates

Figure 11 depicts an amperometric NO/RSNO sensor scheme.

Figure 12 depicts a stability test of copper binding to chelate as determined by atomic absorption spectrum

Figure 13 depicts the catalytic production of NO from GSNO (2mm) for poly(HEMA)-Cu(II)-cyclen polymer films after soaking in plasma/whole blood for different times.

Figures 14A, B and C depict results of acute platelet deposition studies on a stent implanted in a porcine model at 2 hours. Figure 14A is a bare metal stent; Figure 14B is stent with a CVD polymer coating and Figure 14C is a stent with a CVD polymer coating using a nitric oxide generating compound.

### DETAILED DESCRIPTION OF THE INVENTION

### Overview

This disclosure is directed, at least in part, to biocompatible polymers, compositions, devices and coatings that are capable of generating nitric oxide.

### Definitions

For convenience, before further description of the present invention, certain terms employed in the specification, examples, and appended claims are collected here. These definitions should be read in light of the remainder of the disclosure and understood as by a person of skill in the art. Also, the terms "including" (and variants thereof), "such as", "e.g." as used herein are non-limiting and are for illustrative purposes only.

The articles "a" and "an" are used herein to refer to one or to more than one (i.e. to at least one) of the grammatical object of the article. By way of example, "an element" means one element or more than one element.

The terms "biocompatible polymer" and "biocompatibility" when used in relation to polymers are art-recognized. For example, biocompatible polymers include polymers that are neither themselves toxic to the host (e.g., an animal or human), nor degrade (if the polymer degrades) at a rate that produces monomeric or oligomeric subunits or other byproducts at toxic concentrations in the host. In certain embodiments of the present invention, biodegradation generally involves degradation of the polymer in an organism, e.g., into its monomeric subunits, which may be known to be effectively non-toxic. Intermediate oligomeric products resulting from such degradation may have different toxicological properties, however, or biodegradation may involve oxidation or other biochemical reactions that generate molecules other than monomeric subunits of the polymer. Consequently, in certain embodiments, toxicology of a biodegradable polymer intended for in vivo use, such as implantation or injection into a patient, may be determined after one or more toxicity analyses. It is not necessary that any subject composition have a purity of 100% to be deemed biocompatible; indeed, it is only necessary that the subject compositions be biocompatible as set forth above. Hence, a subject composition may comprise polymers comprising 99%, 98%, 97%, 96%, 95%, 90%, 85%, 80%, 75% or even less of biocompatible polymers, e.g., including polymers and other materials and excipients described herein, and still be biocompatible.

To determine whether a polymer or other material is biocompatible, it may be necessary to conduct a toxicity analysis. Such assays are well known in the art. One example of such an assay may be performed with live carcinoma cells, such as GT3TKB tumor cells, in the following manner: the sample is degraded in 1M NaOH at 37 °C until complete degradation is observed. The solution is then neutralized with 1M HCl. About 200 µL of various concentrations of the degraded sample products are placed in 96-well tissue culture plates and seeded with human gastric carcinoma cells (GT3TKB) at 10⁴/well density. The degraded sample products are incubated with the GT3TKB cells for 48 hours. The results of the assay may be plotted as % relative growth vs. concentration of degraded sample in the tissue-culture well. In addition, polymers and formulations of the present invention may also be evaluated by well-known in vivo tests, such as subcutaneous implantations in rats to confirm that they do not cause significant levels of irritation or inflammation at the subcutaneous implantation sites.

The phrase "pharmaceutically acceptable" is art-recognized. In certain embodiments, the term includes compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" is art-recognized, and includes, for example, pharmaceutically acceptable materials, compositions or vehicles, such as a liquid or solid filler, diluent, excipient, solvent or encapsulating material, involved in carrying or transporting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "pharmaceutically acceptable salts" is art-recognized, and includes relatively non-toxic, inorganic and organic acid addition salts of compositions of the present invention, including without limitation, nitric oxide generating agents, excipients, other materials and the like. Examples of pharmaceutically acceptable salts include those derived from mineral acids, such as hydrochloric acid and sulfuric acid, and those derived from organic acids, such as ethanesulfonic acid, benzenesulfonic acid, p-toluenesulfonic acid, and the like. Examples of suitable inorganic bases for the formation of salts include the hydroxides, carbonates, and bicarbonates of ammonia, sodium, lithium, potassium, calcium, magnesium, aluminum, zinc and the like. Salts may also be formed with suitable organic bases, including those that are non-toxic and strong enough to form such salts. For purposes of illustration, the class of such organic bases may include mono-, di-, and trialkylamines, such as methylamine, dimethylamine, and triethylamine; mono-, di- or trihydroxyalkylamines such as mono-, di-, and triethanolamine; amino acids, such as arginine and lysine; guanidine; N-methylglucosamine; N-methylglucamine; L-glutamine; N-methylpiperazine; morpholine; ethylenediamine; N-benzylphenethylamine; (trihydroxymethyl)aminoethane; and the like. See, for example, J. Pharm. Sci., 66:1-19 (1977).

A "patient," "subject," or "host" to be treated by the subject method may mean either a human or non-human animal, such as primates, mammals, and vertebrates.

The term "biocompatible plasticizer" is art-recognized, and includes materials which are soluble or dispersible in the compositions of the present invention, which increase the flexibility of the polymer matrix, and which, in the amounts employed, are biocompatible. Suitable plasticizers are well known in the art and include those disclosed in U.S. Patent Nos. 2,784,127 and 4,444,933. Specific plasticizers include, by way of example, acetyl tri-n-butyl citrate (c. 20 weight percent or less), acetyl trihexyl citrate (c. 20 weight percent or less), butyl benzyl phthalate, dibutyl phthalate, dioctylphthalate, n-butyryl tri-n-hexyl citrate, diethylene glycol dibenzoate (c. 20 weight percent or less) and the like.

As used herein, the term "nitric oxide" encompasses uncharged nitric oxide and charged nitric oxide species, including for example, nitrosonium ion and nitroxyl ion.

The term "metal-ligand complex" refers to a chemical species with at least one ligand capable of coordination with at least one central metal ion.

The term "aliphatic" is an art-recognized term and includes linear, branched, and cyclic alkanes, alkenes, or alkynes. In certain embodiments, aliphatic groups in the present invention are linear or branched and have from 1 to 20 carbon atoms.

The term "alkyl" is art-recognized, and includes saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight chain or branched chain alkyl has 30 or fewer carbon atoms in its backbone (e.g., C₁-C₃₀ for straight chain, C₃-C₃₀ for branched chain), and alternatively, 20 or fewer. Likewise, cycloalkyls have from 3 to 10 carbon atoms in their ring structure, and alternatively about 5, 6 or 7 carbons in the ring structure.

Moreover, the term "alkyl" (or "lower alkyl") includes both "unsubstituted alkyl" and "substituted alkyls", the latter of which refers to alkyl moieties having substituents replacing a hydrogen on one or more carbons of the hydrocarbon backbone. Such substituents may include, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain may themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), -CF₃, -CN and the like. Exemplary substituted alkyls are described below. Cycloalkyls may be further substituted with alkyls, alkenyls, alkoxys, alkylthios, aminoalkyls, carbonyl-substituted alkyls, -CF₃, -CN, and the like.

The term "aralkyl" is art-recognized, and includes alkyl groups substituted with an aryl group (e.g., an aromatic or heteroaromatic group).

The terms "alkenyl" and "alkynyl" are art-recognized, and include unsaturated aliphatic groups analogous in length and possible substitution to the alkyls described above, but that contain at least one double or triple bond respectively.

Unless the number of carbons is otherwise specified, "lower alkyl" refers to an alkyl group, as defined above, but having from one to ten carbons, alternatively from one to six carbon atoms in its backbone structure. Likewise, "lower alkenyl" and "lower alkynyl" have similar chain lengths.

The term "heteroatom" is art-recognized, and includes an atom of any element other than carbon or hydrogen. Illustrative heteroatoms include boron, nitrogen, oxygen, phosphorus, sulfur and selenium, and alternatively oxygen, nitrogen or sulfur.

The term "aryl" is art-recognized, and includes 5-, 6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics." The aromatic ring may be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, -CF₃, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings may be cycloalkyls, cycloalkenyls, cycloalkynyls, aryls and/or heterocyclyls.

The terms ortho, meta and para are art-recognized and apply to 1,2-, 1,3- and 1,4-disubstituted benzenes, respectively. For example, the names 1,2-dimethylbenzene and ortho-dimethylbenzene are synonymous.

The terms "heterocyclyl" and "heterocyclic group" are art-recognized, and include 3-to about 10-membered ring structures, such as 3- to about 7-membered rings, whose ring structures include one to four heteroatoms. Heterocycles may also be polycycles. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring may be substituted at one or more positions with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The terms "polycyclyl" and "polycyclic group" are art-recognized, and include structures with two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms, e.g., three or more atoms are common to both rings, are termed "bridged" rings. Each of the rings of the polycycle may be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, -CF₃, -CN, or the like.

The term "carbocycle" is art recognized and includes an aromatic or non-aromatic ring in which each atom of the ring is carbon. The following art-recognized terms have the following meanings: "nitro" means -NO₂; the term "halogen" designates -F, -Cl, -Br or -I; the term "sulfhydryl" means -SH; the term "hydroxyl" means -OH; and the term "sulfonyl" means - SO₂⁻.

The terms "amine" and "amino" are art-recognized and include both unsubstituted and substituted amines, e.g., a moiety that may be represented by the general formulas: 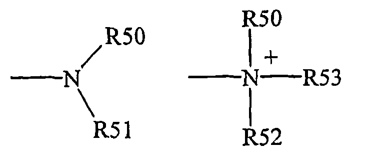 wherein R50, R51 and R52 each independently represent a hydrogen, an alkyl, an alkenyl, -(CH₂)ₘ-R61, or R50 and R51, taken together with the N atom to which they are attached complete a heterocycle having from 4 to 8 atoms in the ring structure; R61 represents an aryl, a cycloalkyl, a cycloalkenyl, a heterocycle or a polycycle; and m is zero or an integer in the range of 1 to 8. In certain embodiments, only one of R50 or R51 may be a carbonyl, e.g., R50, R51 and the nitrogen together do not form an imide. In other embodiments, R50 and R51 (and optionally R52) each independently represent a hydrogen, an alkyl, an alkenyl, or -(CH₂)ₘ-R61. Thus, the term "alkylamine" includes an amine group, as defined above, having a substituted or unsubstituted alkyl attached thereto, i.e., at least one of R50 and R51 is an alkyl group.

The term "acylamino" is art-recognized and includes a moiety that may be represented by the general formula: 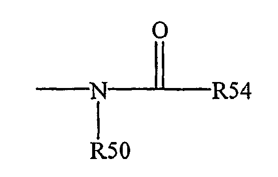 wherein R50 is as defined above, and R54 represents a hydrogen, an alkyl, an alkenyl or - (CH₂)ₘ-R61, where m and R61 are as defined above.

The term "amido" is art recognized as an amino-substituted carbonyl and includes a moiety that may be represented by the general formula: 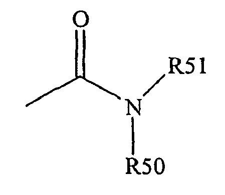 wherein R50 and R51 are as defined above. Certain embodiments of the amide in the present invention will not include imides which may be unstable.

The terms "alkoxyl" or "alkoxy" are art recognized and include an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as may be represented by one of -O-alkyl, -O-alkenyl, - O-alkynyl, -O-(CH₂)ₘ-R61, where m and R61 are described above.

The definition of each expression, e.g. alkyl, m, n, etc., when it occurs more than once in any structure, is intended to be independent of its definition elsewhere in the same structure unless otherwise indicated expressly or by the context.

The abbreviations Me, Et, Ph, Tf, Nf, Ts, and Ms are art recognized and represent methyl, ethyl, phenyl, trifluoromethanesulfonyl, nonafluorobutanesulfonyl, p-toluenesulfonyl and methanesulfonyl, respectively. A more comprehensive list of the abbreviations utilized by organic chemists of ordinary skill in the art appears in the first issue of each volume of the Journal of Organic Chemistry; this list is typically presented in a table entitled Standard List of

### Abbreviations.

Certain monomeric subunits of the present invention may exist in particular geometric or stereoisomeric forms. In addition, polymers and other compositions of the present invention may also be optically active. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of a compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described herein above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

For purposes of this invention, the chemical elements are identified in accordance with the Periodic Table of the Elements, CAS version, Handbook of Chemistry and Physics, 67th Ed., 1986-87, inside cover. The term "hydrocarbon" is art recognized and includes all permissible compounds having at least one hydrogen and one carbon atom. For example, permissible hydrocarbons include acyclic and cyclic, branched and unbranched, carbocyclic and heterocyclic, aromatic and nonaromatic organic compounds that may be substituted or unsubstituted.

The phrase "protecting group" is art recognized and includes temporary substituents that protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed. Greene et al., Protective Groups in Organic Synthesis 2nd ed., Wiley, New York, (1991).

The phrase "hydroxyl-protecting group" is art recognized and includes those groups intended to protect a hydroxyl group against undesirable reactions during synthetic procedures and includes, for example, benzyl or other suitable esters or ethers groups known in the art.

The term "electron-withdrawing group" is recognized in the art, and denotes the tendency of a substituent to attract valence electrons from neighboring atoms, i.e., the substituent is electronegative with respect to neighboring atoms. A quantification of the level of electron-withdrawing capability is given by the Hammett sigma (σ) constant. This well known constant is described in many references, for instance, March, Advanced Organic Chemistry 251-59, McGraw Hill Book Company, New York, (1977). The Hammett constant values are generally negative for electron donating groups (σ(P)=- 0.66 for NH₂) and positive for electron withdrawing groups (σ(P) = 0.78 for a nitro group), σ(P) indicating para substitution. Exemplary electron-withdrawing groups include nitro, acyl, formyl, sulfonyl, trifluoromethyl, cyano, chloride, and the like. Exemplary electron-donating groups include amino, methoxy, and the like.

The term "xylylene" refers to any one of three metameric radicals, such as: 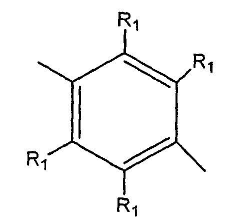 that are derived respectively from the three oriented xylenes. Such xylenes can be optionally substituted with other moieties, for example, R1 can be each independently H, Cl, Br, F, I, NH2, alkyl, alkoxy, alkenyl, aralkyl, alkynyl, or SO₂. A poly(xylylene) comprises at least one xylylene radical or residue.

The term "poly(vinyl)" or vinyl polymer relates to a polymer that is prepared from vinyl monomers, and include any residue or monomer that includes any derivative, substituted or unsubstituted vinyl. An exemplary vinyl polymer includes the residue

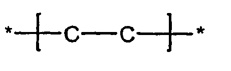

Contemplated equivalents of the polymers, subunits and other compositions described above include such materials which otherwise correspond thereto, and which have the same general properties thereof (e.g., biocompatible, nitric oxide generating), wherein one or more simple variations of substituents are made which do not adversely affect the efficacy of such molecule to achieve its intended purpose. In general, the compounds of the present invention may be prepared by the methods illustrated in the general reaction schemes as, for example, described below, or by modifications thereof, using readily available starting materials, reagents and conventional synthesis procedures. In these reactions, it is also possible to make use of variants which are in themselves known, but are not mentioned here.

### 3. Exemplary Nitric Oxide Generating Agents

A variety of nitric oxide generating agents are contemplated by the present invention. Practitioners of the art will readily appreciate the circumstances under which various nitric oxide agents are appropriate for use in biocompatible coatings.

Nitric oxide generating agents are defined herein to refer to those agents that do not have covalently attached nitric oxide releasing moieties, rather, nitric oxide generating agents are capable of generating nitric oxide when in contact with nitrosothiols, such as those found in bodily fluids and tissues such as blood.

For example, nitric oxide generating agents include metal-ligand complexes. For example, metal-ligand complexes include complexes that have a neutral carrier type ligand with a high metal binding affinity. In some embodiments, such ligands have a high binding affinity for copper. Metal-ligand complexes may have a planar square-type geometry that may provide a minimum amount of steric hindrance to the approach of an electron source to the center metal of the complex so that the metal ion can easily be reduced. Non-limitative examples of such metal-ligand complexes include nitrogen or sulfur donating compounds, such as Nₓ-donor macrocyclic ligands (x=2, 3, 4, 5, 6, 7, 8) such as cyclen, cyclam and their derivatives, and crown ethers and Sy-donor macrocyle-type ligands (y=2, 3, 4, 5, 6, 7, 8). In an embodiment, the metal-ligand macrocycle is a N₄ macrocycle.

Examples of a cyclen complex that can include those metal complexes, include structures such as: 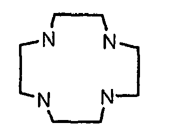 and derivatives of such cyclen ligands .

Metal-cyclam structures include structures such as: 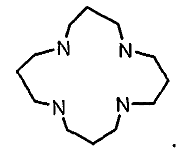 For example, ligands can include cyclam structures that include 1,8 bis(pyridylmethyl)cyclam, 1,11-bis(pyridylmethyl)cyclam, and diooxocyclam ligands and structural isomers thereof. These include multi-amine substrates that can be aromatic or aliphatic.

Exemplary ligands include dibenzo[e,k]-2,3,8,9-tetraphenyl-1,4,7,10-tetraaza-cyclododeca-1,3,7,9-tetraene; dibenzo[e,k]-2,3,8,9-tetramethyl-1,4,7,10-tetraaza-cyclododeca-1,3,7,9-tetraene; dibenzo[e,k]-2,3,8,9-tetraethyl-1,4,7,10-tetraaza-cyclododeca-1,3,7,9-tetraene, and/or salts thereof. Such ligands can be modified to include halogen atoms.

A metal associated with a ligand includes metals and/or metal ions, for example, calcium, magnesium, cobalt, copper, manganese, iron, molybdenum, tungsten, vanadium, aluminum, chromium, zinc, nickel, platinum, tin, ions thereof, and/or mixtures thereof. For example, a metal-ligand complex can include Cu (II)with the ligands noted above. In some embodiments, the metal entity in a metal-ligand complex may be associated with a ligand within the ligand or outside the ligand. A metal-ligand complex can be formed initially or can be formed once a ligand is placed in metal containing tissue or bρdily fluids such as blood.

Without being limited to any theory, nitric oxide generating agents within a composition, when exposed to endogenous or exogenous sources of nitrates, nitrites, or nitrosothiols, and optionally in the presence of reducing agents, generates NO within or at the surface of a composition. It is to be understood that the sources of nitrates, nitrites, nitrosothiols and reducing agents may be from bodily tissues or fluids such as blood, within the composition, within a device, and/or may be injected intravenously or otherwise added or administered to the bodily fluid of interest.

The nitric oxide generating agents contemplated herein may decompose at a temperature that is higher than a typical processing temperature for the manufacture of analyte sensors, and/or at a higher temperature than a nitric oxide releasing agent. For example, the nitric oxide generating agents may decompose at a temperature above about 100 °C, or even above about 125 °C. In an embodiment, the nitric oxide generating agents contemplated by this disclosure are thermally stable.

### 4. Polymers

All of the polymers described herein may be provided or prepared as copolymers or terpolymers.

In certain embodiments, the polymers are comprised almost entirely, if not entirely, of the same subunit. Alternatively, in other embodiments, the polymers may be copolymers, in which different subunits and/or other monomeric units are incorporated into the polymer. In certain instances, the polymers are random copolymers, in which the different subunits and/or other monomeric units are distributed randomly throughout the polymer chain.

In other embodiments, the different types of monomeric units, be they one or more subunits depicted by the subject formulas or other monomeric units, are distributed randomly throughout the chain. In part, the term "random" is intended to refer to the situation in which the particular distribution or incorporation of monomeric units in a polymer that has more than one type of monomeric units is not directed or controlled directly by the synthetic protocol, but instead results from features inherent to the polymer system, such as the reactivity, amounts of subunits and other characteristics of the synthetic reaction or other methods of manufacture, processing or treatment.

In certain embodiments, the subject polymers may be cross-linked. For example, substituents of the polymeric chain, may be selected to permit additional inter-chain cross-linking by covalent or electrostatic (including hydrogen-binding or the formation of salt bridges), e.g., by the use of a organic residue appropriately substituted.

The ratio of different subunits in any polymer as described above may vary. For example, in certain embodiments, polymers may be composed almost entirely, if not entirely, of a single monomeric element. Alternatively, in other instances, the polymers are effectively composed of two different subunits, in which the percentage of each subunit may vary from less than 1:99 to more than 99:1, or alternatively 10:90, 15:85, 25:75, 40:60, 50:50, 60:40, 75:25, 85:15, 90:10 or the like. In other embodiments, in which three or more different monomeric units are present, the present invention contemplates a range of mixtures like those taught for the two-component systems.

In certain embodiments, the polymeric chains of the subject compositions, e.g., which include repetitive elements shown in any of the subject formulas, have average molecular weights ranging from 2000 or less to 10,000,000 or more. Number-average molecular weight (Mn) may also vary widely, but generally fall in the range of 1,000 to 10,000,000. Within a given sample of a subject polymer, a wide range of molecular weights may be present. For example, molecules within the sample may have molecular weights which differ by a factor of 2, 5, 10, 20, 50, 100, or more, or which differ from the average molecular weight by a factor of 2, 5, 10, 20, 50, 100, or more.

One method to determine molecular weight is by gel permeation chromatography ("GPC"), e.g., mixed bed columns, CH₂Cl₂ solvent, light scattering detector, and off line dn/dc. Other methods are known in the art.

Polymers and residues of polymers contemplated for use in disclosed coatings include vinyl polymers, such as N-vinyl pyrrolidone, polypropylene, polystyrene, cinnamyl, poly(vinyl) chloride, acrylates such as poly(methacrylate), poly(methyl methacrylate), poly(acyrl)amide and poly(acrylonitrile). A poly(acyrlate) includes at least one residue of an acrylate, e.g. 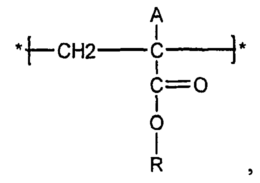 where R may be for example, an alkyl such as methyl, H, a halogen, NH2, or CN. "A" may be for example, any substituent, for example, H or an alkyl such as methyl. In one embodiment, an acrylate may be methacrylate.

### 5. Coatings

Nitric oxide coatings may include a polymer that includes a covalently bonded moiety capable of binding to a metal ion. Such moieties may further include a metal ion, such as described above. A covalently bonded moiety may include a metal-ligand complex, as described herein.

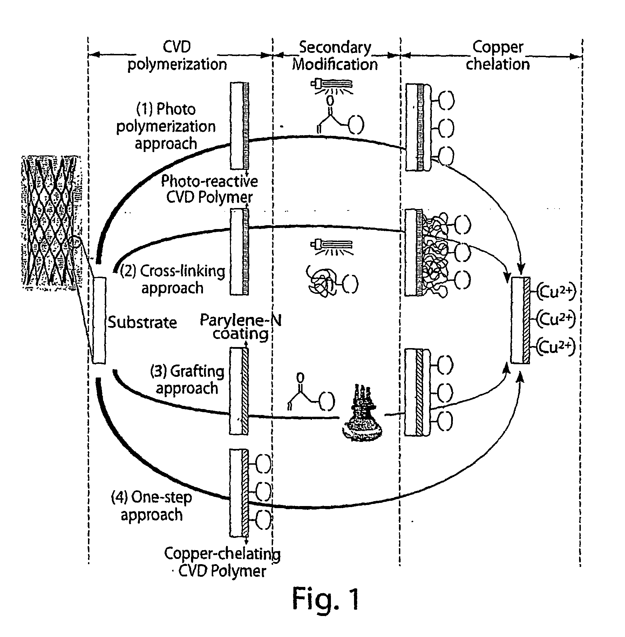

For example, nitric oxide generating biomedical coatings may be made using one or more procedures such as depicted in Figure 1. Such coatings may include a base layer that may enable the adhesion of one or more nitric oxide generating molecular motifs to the substrate surface. Such coatings may also display durability and functionality over sustained time periods. The base layer may consist of a vapor deposited thin film used as it is or in conjunction with a secondary binding layer. In some embodiments, nitric oxide generating molecular motifs may be covalently attached to a vapor deposited base layer. Such an appendant molecular motif may interact with temporary or permanent constituents of the human body to generate locally elevated levels of nitric oxide. For example, nitric oxide generating biomedical coatings may be applied to the surface of a biomedical device that includes an intra- or extracorporeal biomaterial. These biomaterials may comprise a single material type, e.g. a metal, alloy, ceramic, or polymer, or hybrid structures thereof.

Binding or association of a nitric oxide generating molecular motifs to a base layer may be achieved by employing vapor based coatings in a variety of settings.
Suitable methods include, but are not necessarily limited to (1) photopolymerization of monomers on the CVD coating, (2) photochemical cross-linking of a NO generating matrix to the CVD adhesion layer using the photo-definable CVD coatings, (3) chemical grafting of a NO- a generating matrix from the surface of the CVD coating, and (4) direct metal ion-binding poly-p-xylylenes. Approach (3) may involve formation of hydrogel coatings that possess Cu(II)-complex sites that may be chemically interconnected to an adhesion layer. Such CVD methods produce a coating that may be substantially free of organic solvents. Figure 1 illustrates pictorially the four methods based on CVD technology. Photo-definable CVD coatings include poly-p-xylylenes (PPX).

For example, photodefinable poly-p-xylylenes may be subsequently modified via polymerization/cross-linking with copper-binding monomers and hydroxyethylmethacrylate (HEMA). This may be a vapor-deposited polymer with associated fidelity, stability, and flexibility characteristics.

Photochemical immobilization chemistry may be utilized with such vapor deposited polymers. The concentration and ratio of a metal-binding monomer and a polymer or residue, such as a photoactivate residue or monomer, e.g. acrylates such as methyacrylates and HEMA; cinnamyl or cinnamoyls; benzophenone and radicals and derivatives thereof, and perfluorophenyl azides can be controlled to design coatings with different metal ion-ligand loadings. Without being bound by any theory, such vapor deposited monomer and polymers can yi eld a ratio of nitric oxide generating agents to area of coating that may be substantially optimal to generate nitric oxide from such a coating.

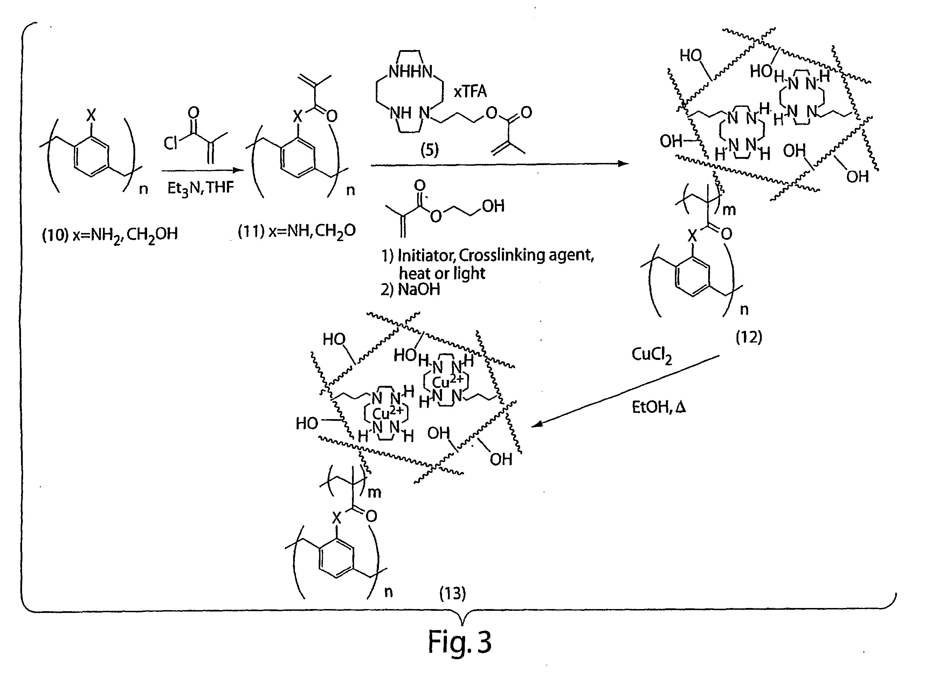

Alternatively, preformed metal-containing polymethacrylates may be tethered to an underlying CVD surface by utilizing light induced CH abstraction. Surface loading may be systematically varied based on different binding site densities and the molecular weight of the polymers. For example, a linear or lightly crosslinked polymethacrylate with appended metal-complexes may be designed in accordance with the chemistry in Figure 3.

In some embodiments, the polymers may be dissolved in a solvent, for example, water or alcohol, prior to a photoimmobilization.

Solutions of the polymers can then be applied to a layer, such as a layer of benzoyl-PPX, for example, on for example, stainless steel devices or disks using dip or spray coating techniques, for example, to create a coating thereon.

In an embodiment, subsequent irradiation with 365 nm light of the surface may cause spontaneous CH abstraction, as shown in Figure 3, from the polymethacrylate/copper(II) cyclen polymer, thereby creating a stable linkage between the pre-made polymer and the CVD surface.

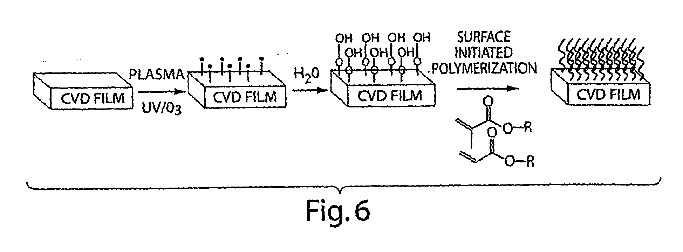

Polymers such as poly-p-xylylenes may be modified via "grafting-from-surface" technique. This technique may utilize a metal ion-chelating methacrylates monomer and HEMA to grow polymer chains from the surface (e.g., brush-like structures). This method comprises a surface-initiated polymerization that may create a non-cross-linked coating architecture. Parylene coatings may be used as an interface after activation, by for example, ultraviolet radiation or ozone, may act as initiator sites for graft-co-polymerization, thus creating linear chains of polymethacrylates with appended metal ion binding sites originating from the surface CVD layer. In this approach, the polymethacrylate/Cu(II) cyclen structures are grown from the surface of the CVD as linear polymer strands. Figure 6 illustrates the resulting "brush-like" structure with minimal chain-chain cross-links. The polymer may grow directly from the surface. Metal ion sites may be appended at varying spacing distances. This distance may vary as a function of the ratio of the monomers used in the reaction.

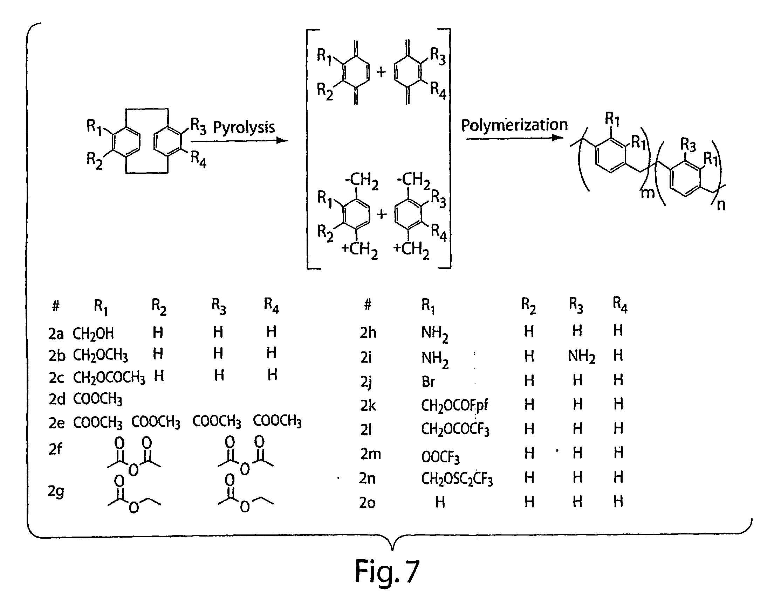

In another embodiment, synthesis of metal ion binding polyenes such as poly-p-xylylenes may use CVD polymerization to deposit metal-binding poly-p-xylylenes. For example, a Cu(II)-binding [2.2]paracyclophane species, for example those shown in Figure 7, may be synthesized and then polymerized in situ via a CVD process. This approach may not require any further polymer-analogue modifications to create metal ion ligand coatings.

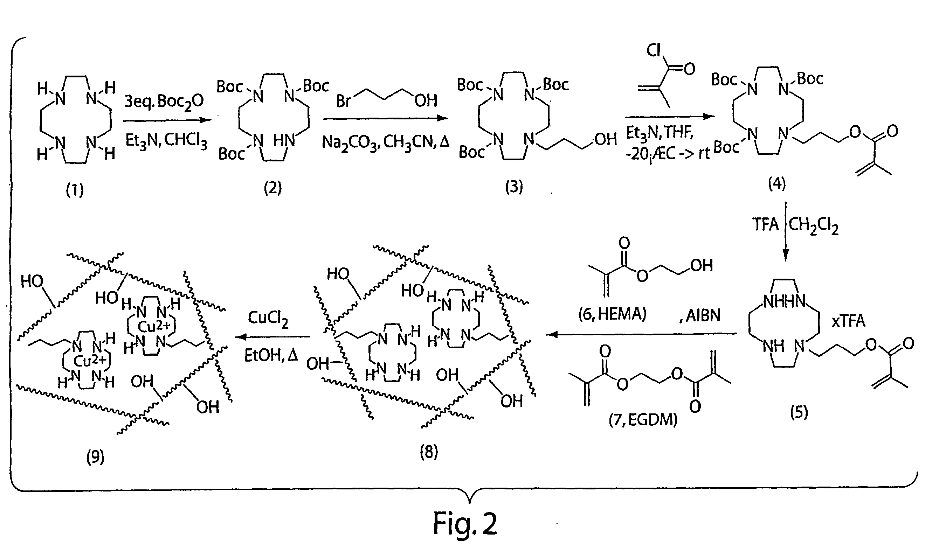

For example, poly(4-benzoyl-p-xylylene-co-p-xylylene) (benzoyl-PPX) may be used as the CVD adhesion layer. A hydrogel containing a Cu(II)-binding ligand can then be attached to the surface a metal, such as a stainless steel. The solution containing the cyclen monomer is photopolymerized in the presence of 2-hydroxyethylmethacrylate (HEMA), and polyethylene glycol dimethacrylate (PEG-dMA). The coated disks can then be exposed to UV radiation to photoactivate the CVD polymer and concomitantly create a crosslinked hydrogel attached to the CVD polymer as shown in Figures 2 and 3.

Coatings of the instant disclosure are , used on medical devices, and in some embodiments, on a metal surface or layer of a medical device. Such medical devices include, for example, an intravascular medical or delivery device, such as vascular catheters, (e.g. balloon catheter, an injection catheter, and an infusion catheter), a stent, a stent graft, vascular grafts, guide wires, balloons, filters (for example, vena cava filters), aneurysm fillers (including for example Guglielmi detachable coils), intraluminal paving systems, urinary catheters, valves, stets, shunts, pacemaker leads, implantable defibrillator, adventitial wrap, or a distal protection device.

Coatings contemplated herein are between 10 nm to 2000 nm, between 20 nm and 200 nm, or even 20 nm to 100 nm.

In addition to the nitric oxide generating agent, the subject coatings may contain or be pendantly bonded to other therapeutic agents. Any therapeutic agents in a subject composition may vary widely with the purpose for the composition. The term therapeutic agent includes without limitation, medicaments; vitamins; mineral supplements; substances used for the treatment, prevention, diagnosis, cure or mitigation of disease or illness; or substances which affect the structure or function of the body; or pro-drugs, which become biologically active or more active after they have been placed in a predetermined physiological environment. Compositions contemplated by this disclosure can include one or more nitric oxide releasing agents alone or in combination with one or more nitric oxide generating agents.

Suitable therapeutic agents useful for the coatings and devices disclosed herein, include, but are not limited to, antithrombogenic agents (such as, for example, heparin, covalent heparin, hirudin, hirulog, coumadin, protamine, argatroban, D-phenylalanyl-L-poly-L-arginyl chloromethyl ketone, and the like); thrombolytic agents (such as, for example, urokinase, streptokinase, tissueplasminogen activators, and the like); fibrinolytic agents; vasospasm inhibitors; potassium channel activators (such as, for example, nicorandil, pinacidil, cromakalim, minoxidil, aprilkalim, loprazolam and the like); calcium channel blockers, antihypertensive agents; anti-infective agents including antiviral agents, antimicrobial agents and antifungal agents, antimicrobial agents or antibiotics (such as, for example, adriamycin, and the like); antiplatelet agents (such as, for example, aspirin, ticlopidine, a glycoprotein IIb/IIIa inhibitor, surface glycoprotein receptors and the like); antimitotic, antiproliferative agents or microtubule inhibitors (such as, for example, taxanes, colchicine, methotrexate, azathioprine, vincristine, vinblastine, cytochalasin, fluorouracil, adriamycin, mutamycin, tubercidin, epothilone A or B, discodermolide, and the like); antisecretory agents (such as, for example, retinoid, and the like); remodelling inhibitors; antisense nucleotides (such as, for example, deoxyribonucleic acid, and the like); anti-cancer agents (such as, for example, tamoxifen citrate, acivicin, bizelesin, daunorubicin, epirubicin, mitoxantrone, and the like); steroids (such as, for example, dexamethasone, dexamethasone sodium phosphate, dexamethasone acetate, β-estradiol, and the like); non-steroidal antiinflammatory agents (NSAID); COX-2 inhibitors; 5-lipoxygenase (5-LO) inhibitors; leukotriene A4 (LTA4) hydrolase inhibitors; 5-HT agonists; HMG-CoA inhibitors; antineoplastic agents, thromboxane inhibitors; decongestants; diuretics; sedating or non-sedating anti-histamines; inducible nitric oxide synthase inhibitors; opioids, analgesics; Helicobacter pylori inhibitors; proton pump inhibitors; isoprostane inhibitors; vasoactive agents; beta.-agonists; anticholinergic; mast cell stabilizer; immunosuppressive agents (such as, for example cyclosporin, rapamycin, everolimus, actinomycin D and the like); growth factor antagonists or antibodies (such as, for example, trapidal (a PDGF antagonist), angiopeptin (a growth hormone antagonist), angiogenin, and the like); dopamine agonists (such as, for example, apomorphine, bromocriptine, testosterone, cocaine, strychnine, and the like); radiotherapeutic agents; heavy metals functioning as radiopaque agents (such as, for example, iodine-containing compounds, barium-containing compounds, gold, tantalum, platinum, tungsten, and the like); biologic agents (such as, for example, peptides, proteins, enzymes, extracellular matrix components, cellular components, and the like); angiotensin converting enzyme (ACE) inhibitors; angiotensin II receptor antagonists; renin inhibitiors; free radical scavengers, iron chelators or antioxidants (such as, for example, ascorbic acid, alpha tocopherol, superoxide dismutase, deferoxamine, 21-aminosteroid, and the like); sex hormones (such as, for example, estrogen, and the like); antipolymerases (such as, for example, AZT, and the like); antiviral agents; photodynamic therapy agents (such as, for example, 5-aminolevulinic acid, meta-tetrahydroxyphenylchlorin, hexadecafluoro zinc phthalocyanine, tetramethyl hematoporphyrin, rhodamine 123, and the like); antibody targeted therapy agents (such as, for example, IgG2 Kappa antibodies against Pseudomonas aeruginosa exotoxin A and reactive with A431 epidermoid carcinoma cells, monoclonal antibody against the noradrenergic enzyme dopamnine beta-hydroxylase conjugated to saporin, and the like); and gene therapy agent. Therapeutic agents may further include antiproliferative agents, such as, for example, taxanes; steroids such as, for example, dexamethasone, β-estradiol, immunosuppressive agents, such as for example, rapamycin, everolimus, actinomycin D, NSAIDs, such as, for example, acetaminophen, aspirin, diclofenac, ibuprofen, ketoprofen, naproxen and the like.

### 6. Exemplary Methods for Treating Unwanted Inflammatory Response

The devices disclosed herein may prevent or ameliorate an unwanted inflammatory response in a patient when such a device is implanted in a patient. As contemplated by the present invention, the nitric oxide generating agents bound to a coating generate nitric oxide in an amount sufficient to deliver to a patient a therapeutically effective amount of nitric oxide as part of a prophylactic or therapeutic treatment. As also contemplated herein, the coatings on a device may be are formulated for sustained or extended generation of a therapeutically effective amount of nitric oxide without replenishment of the nitric oxide generating agents so that coating that is sufficiently thin may be sufficient to treat any unwanted inflammatory response adequately. Unwanted inflammatory responses include restonsis and thrombosis including acute and subacute thrombosis.

The efficacy of treatment with the subject device and/or coatings may be determined in a number of fashions. For example, a comparison of the different devices or coatings may be based on the effectiveness of the coatings or devices, with a subject coating being better, or 50%, 100%, 150%, 200%, 300% more effective, than by another method using no nitric oxide generating agent, using a nitric oxide donating agent, and/or using a different coating process.

### 7. Exemplification

The invention having been generally described, it will be more readily understood by reference to the following examples which are included merely for purposes of illustration of certain aspects and embodiments of the present invention and are not intended to limit the invention in any way.

All reagents and solvents were purchased from Aldrich or Fisher Chemical Co. Unless otherwise noted, they were used without further purification.

### Example 1 Synthesis of poly(Cu(II)-cyclen-N-3-propyl methacrylate-co-2-hydroxyethyl methacrylate) hydrogel (9).

As shown in Figure 2, 3Boc-cyclen 2 is prepared and purified by from commercially available cyclen **1**. A mixture of compound **2** and 3-bromopropanol is heated to 80 °C in the presence of sodium carbonate in acetonitrile overnight to afford 3Boc-cyclen-N-3-propanol 3 (67% over 2 steps after purification with silica gel chromatography). To introduce a polymerizable group onto compound 3, methacryloyl chloride is slowly added to a solution of compound 3 and triethylamine in dry THF at - 20 °C. After 1h, the mixture is allowed to warm to room temperature. 3Boc-cyclen-N-3-propyl methacrylate monomer 4 is obtained after column chromatography with silica gel (59% yield). After deprotection of compound 4 with trifluoroacetic acid (TFA), 7.6 mol % of a crude solid compound 5 is polymerized with 2-hydroxyethyl methacrylate (6, HEMA, purified by distillation before using), and 2 wt % of ethylene glycol dimethacrylate (7, EGDM) in the presence of 0.4 wt % of 2,2'-azobisisobutyronitrile (AIBN) in methanol on a glass slide at 65 °C overnight. Removal of unreacted starting materials and low molecular weight compounds is achieved by removing the film from the glass and heating to 80-90 °C in ethanol for 3-4 h. After cooling to room temperature, the resulting hydrogel is filtered and washed with ethanol, then stirred and washed with several portions of dilute NH₄OH. Further washing with deionized water and ethanol affords poly(eyelen-N-3-propyl methacrylate-co-2-hydroxyethyl methacrylate) hydrogel (8). Refluxing of hydrogel 8 with hydrated CuCl₂ in ethanol enables the incorporation of Cu(II) ion into the hydrogel. Again, after extensive washing with ethanol and deionized water to remove free copper ions physically or weakly bound to the polymer backbone, poly(Cu(II)-cyclen-N-3-proply methacrylate-co-2-hydroxyethyl methacrylate) hydrogel 9 is obtained. The copper content of dried hydrogel 9 is 2.2% (theoretical value, 2.8%) as determined by ICP-MS (Inductively Coupled Plasma Emission-Mass Spectrometry). The swelling ratio of the hydrogel is 2.0 as determined by q, the ratio of the weight of swollen state and the weight of dried state.

### Example 2 Synthesis of poly(2-hydroxyethyl methacrylate) hydrogel 11.

Poly(2-hydroxy methacrylate) hydrogel 11, a blank hydrogel, is synthesized via 0.8 wt % AIBN, 4 wt % of EGDM, and HEMA and copper ion incorporation and purification using the same methods employed for preparation of hydrogel 9.

### Example 3 Nitric oxide generation of blank hydrogels, 8 and 11 from S-nitrosoglutathione (GSNO) and glutathione (GSH).

To ensure that the NO generation is induced only from copper ions chelated by cyclen, not ions adsorbed to the polymer backbone, two blank hydrogels, **8** and **11** are investigated for their catalytic NO generation from GSNO and a reducing equivalent, GSH in the presence of a strong copper chelator, EDTA in deoxygenated PBS buffer (pH=7.4). The hydrogels, 8 and 11 are soaked separately for 1 d and bubbled with nitrogen gas for 30 min in PBS buffer (pH=7.4) prior to experiments. Hydrogel 8 does not generate NO at all, indicating that copper ions are required for NO generation. The initial immersion of hydrogel 11 into the reaction solution shows a small NO flux, but spontaneously decreases to the baseline; hence the next trial of the same polymer does not generate NO at all. The polymer matrix itself incorporates a small amount of copper ions without the cyclen moiety, causing slight NO generation upon the first trial; however the amounts of copper ions physically or weakly bound to the polymer backbone would be too small to yield sequential catalytic NO generation.

### Example 4 Catalytic NO generation of hydrogel 9 from GSNO and GSH. As shown in Figure 1 (B, C, and D)

Hydrogel 9 catalytically generates NO under the same reaction conditions as used for blank hydrogels 8 and 11. Pre-experiment treatments of hydrogel 9 are the same as hydrogels 8 and 11. Upon the addition of hydrogel 9 into the reaction solution, relatively large amounts of NO are generated followed by a decrease, eventually reaching a steady-state flux. When hydrogel 9 is removed from reaction cell, the NO flux decreases to the baseline quickly. Subsequent immersion/removal cycles of the same piece of hydrogel 9 demonstrate that the hydrogel can reversibly achieve a steady-state NO flux. The same piece of hydrogel 9 in a fresh reaction solution also generates the nearly same steady-state levels of NO without a transient flux. In fact, the same piece of hydrogel 9 soaked in PBS for 3 days and 6 days exhibits nearly the identical pattern of NO fluxes during several separate experiments, indicating that hydrogel 9 has stable catalytic activity to generate NO from GSNO and GSH at physiological pH.

### Example 5 Catalytic NO generation of hydrogels 9 from nitrite and ascorbate.

Hydrogel 9 also catalytically generates NO from inorganic nitrite (NaNO₂ in the presence of reducing agent, ascorbate, and EDTA at physiological pH. However, there is clearly a different behavior of NO generation observed in the case of the nitrite and ascorbate solution. It takes more than 20 min to reach a similar steady-state NO flux in much higher concentrations of nitrite (1 mM) and ascorbate (100 µM) compared to approximately 5 min in the case of GSNO (10 µM) and GSH (30 µM) solution. Furthermore, there is no transient NO generation during the first cycle as is observed when employing GSNO. It should be noted that hydrogel 9 yields almost the same levels of NO flux regardless of the different EDTA concentration (10 and 100 µM), although it is known that the concentrations of EDTA in human plasma are negligible (less than 0.05 µM).

### Example 6

To prepare nitiric oxide generating coatings, Cu(II)-containing hydrogels are covalently linked to the CVD polymer by photoactivation in the presence of monomers containing Cu(II)-ligands and a photoinitiator. These polymerize at the surface and concomitantly anchor to the CVD layer. After creating the polymerized film, Cu(II) ions are loaded by reacting film with copper(II) chloride. Using poly(4-benzoyl-p-xylylene-co-p-xylylene) (benzoyl-PPX) as the CVD adhesion layer, a hydrogel containing a Cu(II)-binding ligand (e.g., cyclen) is attached to the surface of the stainless steel disks (as an initial model for stent surfaces). The solution containing the cyclen monomer is photopolymerized in the presence of 2-hydroxyethylmethacrylate (HEMA), and polyethylene glycol dimethacrylate (PEG-dMA). The coated disks are exposed to UV radiation to photoactivate the CVD polymer and concomitantly create a crosslinked hydrogel attached to the CVD polymer as shown in Figure 1, Method 1. The disks are repeatedly washed with DI-water to remove any excess monomer solution and are characterized using infrared spectroscopy (FT-IR/ATR) to determine the relative composition of the monomers in the hydrogel layer. To incorporate the Cu(II)-sites, the polymer-coated disks are exposed to a solution containing copper (II) chloride for up to 3 h. After this exposure, the surfaces are washed thoroughly then analyzed for copper content using XPS or atomic absorption (after dissolving a given mass of polymer film in strong acid) to confirm that bound Cu(II) levels correlate with the amount of cyclen monomer used in the reaction mixture. The NO generating capability of such modified surfaces is determined using standard chemiluminescence detection.

### Example 7: Fabrication of polymethacrylate/Cu(II)-cyclen hydrogels on the surface of a benzoyl-PPX CVD layer deposited on stainless steel.

he photoactive CVD adhesion layer is first obtained by CVD polymerization of the precursor, benzoyl-PPX, using a CVD installation consisting of a sublimation zone, pyrolysis zone and deposition chamber. The precursor is placed in the sublimation zone and the stainless steel disk is placed on the sample holder at 10 °C. The precursor is slowly sublimed at a temperature of 100 °C and a low pressure of 50 µbar. The carrier gas-argon (flow rate of 20 sccm) then carries the precursor into the pyrolysis zone which is at a temperature of 670 °C. Next, a solution containing 31.5 wt. % cyclen-derivatized methacrylate 66 wt. % HEMA and 2.5 wt. % polyethylene glycol dimethacrylate (PEG-dMA) is applied onto stainless steel disks coated with a benzoyl-PPX CVD polymer. These disks are then exposed to UV radiation for 30 min to photoactivate the polymer and create a crosslinked hydrogel on the CVD polymer.

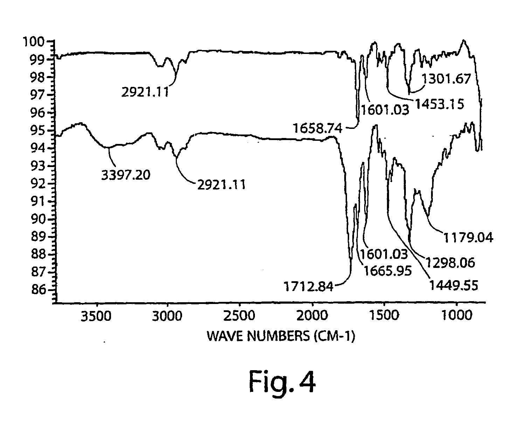

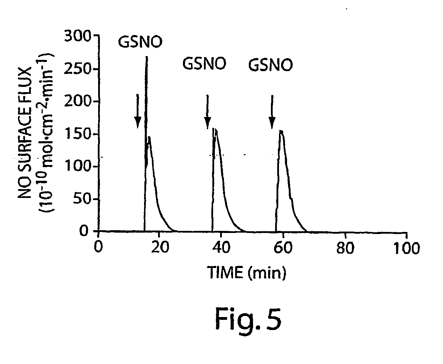

Figure 4 shows an infrared (IR) spectra of the CVD polymer and the polymethacrylate/Cu(II)-cyclen on CVD coatings on the stainless steel substrates. IR spectroscopy confirms the presence of the carbonyl group characteristic of the methacrylates, as indicated by the strong signal at 1712 cm⁻¹ in (B). The broad peak seen at 3397 cm⁻¹ shows the hydroxyl groups present in 2-hydroxyethyl methacrylate (HEMA) which are absent in the polymer and thus the peak is not observed in (A). Figure 5 illustrates the ability of the coating to generate NO, as measured by chemiluminescence, from physiological S-nitrosoglutathione and glutathione. In three separate injections, the CVD/polymethacrylate/Cu(II)-cyclen coatings were able to convert the S-nitrosoglutatione to NO under physiological conditions (37 °C, pH 7.4).

It is also possible to substitute polyethylene glycol methacrylates (PEGMA) of variable molecular weights for HEMA. In addition, the hydrophobilicity/hydrophilicity of the hydrogel layers may be altered by substituting isodecylmethacrylate for HEMA or PEGMA in the reaction process.

### Example 8:

In this methodology, pre-made linear or lightly crosslinked polymethacrylate are prepared with appended Cu(II)-complexes in accordance with the chemistry in Figures 2 and 3. The polymers are disolved in water or alcohol prior to the photoimmobilization. Solutions of the polymers are applied to a benzoyl-PPX layer on stainless steel disks using dip or spray coating techniques. Subsequent irradiation of about 365 nm light of the surface causes spontaneous CH abstraction from the polymethacrylate/copper(II) cyclen polymer, thereby creating a stable linkage between the pre-made polymer and the CVD surface. This aspect of the invention is schematically illustrated in Figure 1, Method 2. The resulting coatings are characterized by FTIR/ATR, XPS, and for NO generation. The initial pre-made polymethacrylate%opper (II) cyclen polymers are varied in terms of their initial copper content via controlling monomer ratios and in terms of the molecular weight of the pre-made polymers as determined by gel permeation chromatography (GPC).

### Example 9:

The polymethacrylate/Cu(II) cyclen structures are grown from the surface of the CVD as linear polymer strands. This is schematically illustrated in Figure 6. To achieve surface grafting, radicals are created on the poly-p-xylylene coatings subsequent to CVD polymerization. Such meta-stable radicals are formed in polymer surfaces via (a) low-temperature plasma treatment or (b) UV/ozone treatment. The meta-stable radicals are used as surface-bond initiator sites for the thermally induced radical polymerization of the methacrylates. As illustrated in Figure 1, and Figure 6, this design yields a "brush-like" structure with the polymer being grown directly out from the surface, with Cu(II) sites appended at varying spacing distances, depending on the ratio of the monomers used in the reaction.

### Example 10:

Nitric oxide generating coatings are fabricated by first removing oxygen from the reaction system by vacuuming the reactor for 5 min followed by flowing argon gas to it for another 5 min. HEMA and a degassed solution containing DI water and ethanol in a ratio of 4: 1 (DI water/ethanol) are then added to the reaction vessel and stirred. This grafting solution is heated up to 65 °C. During this time, the stainless steel disks are placed on the loading tray of a Jelight 342 UV Ozone Cleaner. The disks are arranged so that the side to be activated is 5mm below the UV lamp to maximize exposure to UV Ozone. The disks are activated for 20 minutes and then placed into the reactor. The grafting solution is heated to the reaction temperature of 80 °C. Grafting is conducted for 3 hours. Once the reaction is complete, the heat source is turned off to let the solution cool down for 30 minutes. The disks are removed from the reaction vessel and sonicated in DI water for 15 minutes. Argon gas is blown over the disks to dry them. By using the same methacrylated derivative of cyclen (compound **5** in Figure 2), it is possible to prepare linear co-polymers that contain different amounts of Cu(II) sites by controlling the ratio of monomers used in the grafting procedure. Other monomers may be used to optimize surface properties of the appended polymers/copper (II) cyclen structures (e.g., wettabilitiy, resistance to protein fouling, stability, etc.), such as PEGMA and isodecylmethacrylate.

### Example 11 Preparation of functionalized [2.2]paracyclophanes that have side groups that can chelate copper.

The CVD polymerization follows in principal the method described in example 6. During the CVD polymerization, the solid paracyclophane (dimer) is first vaporized at about 150 °C in a 0.2 mbar vacuum and the resulting gas is then heated to 600 - 800 °C to yield the monomer para-xylylene. In the last step, the monomer gas is adsorbed as it polymerized on the substrate at temperatures around 30 °C. A range of [2.2] paracyclophanes exist that establish potential candidates for this approach. Successful candidate structures include diamino [2.2]paracyclophanes. Other structures include dithiol [2.2]paracyclophanes and di(aminomethyl) [2.2]paracyclophanes.

### Example 12 Preparation of functionalized [2.2]paracyclophanes that have side groups which can be converted into groups that can chelate Cu(II).

The CVD polymerization followed in principal the method described in example 6. However, the binding of Cu(II)-ligands inherently to a PPX coating is achieved by a two-step procedure where the functionalized PPX were prepared first and the functional groups are used to attach the prepared copper chelating groups.

### Example 13

Physical and chemical properties of the CVD-coatings are determined from coated stainless steel foils. Routine characterization of chemical and physical properties of CVD-coated surfaces include elementary analysis, X-ray photoelectron spectroscopy, infrared spectroscopy, and scanning electron microscopy (SEM). The XPS results, which reveal composition of the outermost 10 nm, are compared to bulk analysis via elemental analysis. In parallel, depth profiling via XPS is evaluated. The experimental result is compared to theoretical values calculated assuming the composition of [2.2]paracyclophane and polymer is identical.

### Example 14

Deposited in thin films, CVD-coatings are evaluated for their adhesion properties on relevant substrate materials. Adhesion of the coating to a substrate is examined by pressing a 1 cm² area of a Scotch tape onto the polymer coating. After peeling off the tape, the sample is examined by optical microscopy and infrared spectroscopy. The CVD polymerization is optimized to ensure the films are mechanically and chemically intact. Furthermore, the polymer coating is tested for stability in distilled water or ethanol for four weeks. Atom adsorption spectroscopy is utilized to determine the copper(II) binding capacity of the BCC's and electrochemical measurements in animal blood to assess the generation of nitric oxide via catalytic copper(II) reactions from endogenous RSNO species.

### Example 15

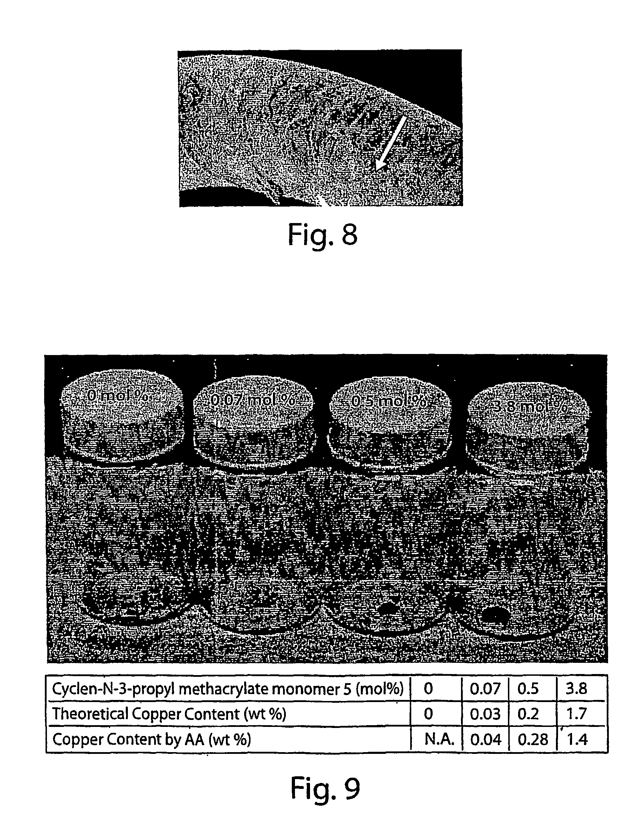

Stability studies are conducted with stent devices. Figure 8 shows an SEM image that exemplifies the effects of such rigid protocols onto the polymer coatings. To minimize the mismatch in mechanical properties and to prevent delamination, a film thicknesses below 200 nm is utilized. SEM is a powerful method to inspect this kind of disinitegrities. A further factor that can interfere with the stability of the polymer coatings, the functional groups used for anchoring, and the integrity of the Cu(II)-complex, is the sterilization process. Sterilization involves harsh treatments that may be a potential cause of functional loss. Two established methods are utilized; ETO sterilization and ion beam sterilization, to study the influence on the functional coatings deposited on stents and without drug loading. Sterilization methodsthat result in minmal changes in morphology and chemical composition are used to sterilize the biocatalytic copper coatings.

### Example 16 Cytotoxicity studies

Bio-catalytic copper coatings are prepared and ranked and the candidates with the best NO generating capability and least leaching of copper are further examined for cytotoxicity and preliminary in vivo biocompatibility. Coatings on small pieces of stainless steel substrates, or stainless steel tubings are inserted into test animals to allow mimicing of the actual layered stent structures that will ultimately be prepared. Initially, standard ISO-10993 in vivo testing protocols for systemic toxicity, irritation sensitization, and inflammatory/fibrotic responses to implant healing of the various CVDbased copper(II) coatings in small animal models are carried out. The in vivo studies address cytotoxicity, systemic toxicity, sensitization, and inflammatory/fibrotic responses to the various bio-catalytic coatings candidates.

### Example 17 Cytotoxicity studies:

Potential bio-catalytic coatings are assessed for their cytotoxic responses in human umbilical endothelial vein cells (HUVECs) and umbilical artery smooth muscle cells (UASMC), utilizing published ISO 10993-5 testing procedures for elution and agar diffusion methods aswell as assays to monitor cell proliferation and migration. Cell migration is assyaed on coated stainless steel disks using the well-established wounded cell monolayer migration assay. Cell proliferation is assayed using ABSOLUTE-S SBIP Cell Proliferation Assay (Molecular Probes) and flow cytometry.

### Example 18 Systemic toxicity studies:

Following the cytotoxicity testing, the total number of probable bio-catalytic coatings are evaluated for their potential toxic effects on organs and tissues remote to the site of contact. The tests are carried out according to the standards described in ISO 10993-11. Briefly, the bio-catalytic coatings and their components are equilibrated in saline and vegetable oil at 37°C for 24 h. Systemic toxicity is tested by intravenous (IV) or intraperitoneal (IP) injection of these solution preparations into groups of five mice per condition. Control mice are injected with either saline or vegetable oil alone. The mice are monitored during the subsequent three days for adverse responses, such as convulsions or prostration. Bio-catalytic coatings that do not pass the systemic cytotoxicity tests are eliminated.

### Example 19 Sensitization studies:

Immune sensitization to the bio-catalytic coatings are conducted according to ISO 10993-10 standards. Fluid extracts of each of the bio-catalytic coatings and its components are prepared in saline and vegetable oil at 37°C for 24 h. Injections of each extract and an adjuvant designed to induce an immune response occur in separate groups of three guinea pigs per condition. Control guinea pigs are injected with the adjuvant plus either saline or vegetable oil. Following the injections, a two week incubation period is observed to allow for the development of a delayed response at which point each guinea pig is challenged by a final exposure to the extract to which they were sensitized previously. A sample of the extract is applied topically to an area of skin devoid of hair. Each application site is monitored for adverse reactions such as redness and swelling. The coatings are tested for inflammation and fibrotic responses.

### Example 20 Inflammatory and fibrotic response to implantation:

Local inflammatory and fibrotic responses to short term (1, 4, and 28 week) implantations is assesed by intramuscular implantation into albino rabbits according to ISO 10993-6 standards. Bio-catalytic coatings adhered to either metal discs or strips are implanted using 15-19 gauge needles into the paralumbar musculature on one side of the back at three separate sites. Three control substances are implanted into the musculature on the opposing side of the back. At the conclusion of each time point the animal is sacrificed, tissue explanted, and grossly examined for fibrotic capsule formation. Following this examination, the explanted tissue is processed by scanning electron microscopy (SEM) or histologic light microscopy.

### Example 21 Synthesis

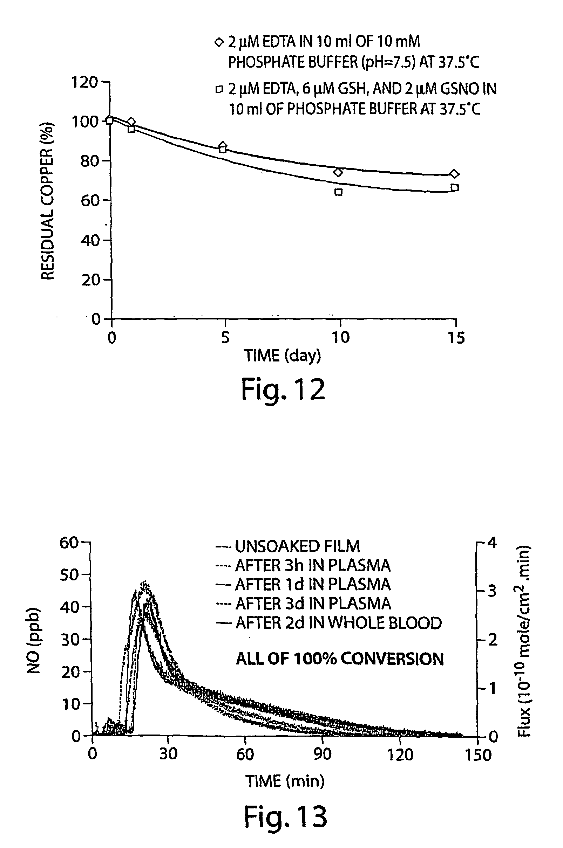

Compound 5 is synthesized and confirmed by NMR and Mass-Spec. This monomer is co-polymerized (thermally) with 2-hydroxyethylmethacrylate (HEMA) (at varying mole ratios) and a crosslinking agent (EGDM). The Boc protecting groups on the cyclen structures are then removed, and the resulting polymers are washed with NaOH solution, pH 10-11, then water and EtOH, and then reacted with CuCl₂ in the presence of EtOH and heat to generate the corresponding Cu(II) complex The amount of Cu(II) is measured by atomic absorption and correlates with the amount of cyclen monomer used in the original. After extensive washing with water, a small piece of one of the polymeric materials (4.6 mg polymer made with 3.8 mol% 6) is placed into 2 ml of PBS buffer containing 2µM GSNO, 5 µM GSH, and 10 µM EDTA. As illustrated in Figure 13, immediate NO generation is observed. Figure 12 further illustrates that NO generation is maintained even after soaking pieces of the catalytic polymer film in sheep plasma for varying times up to 3 days. In this latter experiment, the film is left in the solution and all theGSNO (2 µM) is converted to NO over time (90 min).

### Example 22 Leaching

Leaching of the Cu(II) ions from the poly(HEMA) materials after prolonged exposure to PBS buffer containing EDTA and GSNO (at 2 µM and GSH (6 µM) is assessed. Even with EDTA and GSH present, the level of copper within polymeric films prepared with such poly(HEMA)-cyclen materials is 70% of the initial levels after 2 week (Figure 13, with repeated equilibrations with fresh buffer each day at 37 °C). The very strong binding constant for EDTA binding to Cu(II) is 1023 M⁻¹.

### Example 23 Sensor

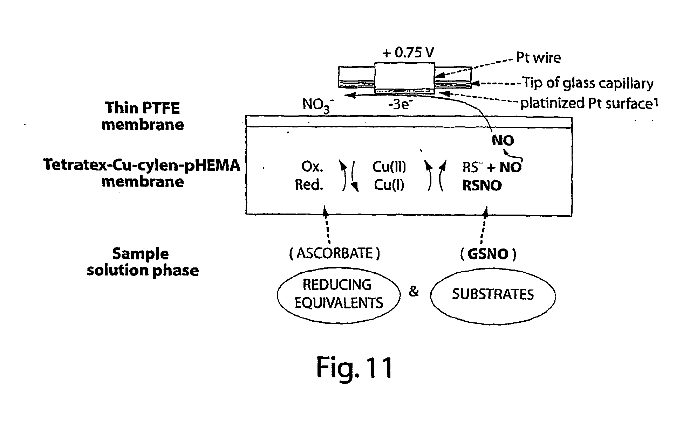

NO-generating capability of fresh blood from the same animals in which the oxygensensing catheters will be placed intravascularly can be assessed. An electrochemical sensor that is capable of carrying out this analytical task is illustrated in Figure 11. This amperometric RSNO sensor is used to probe the variability in levels of reactive RSNO Species (e.g.,CysNO, GSNO, etc.). The sensor exhibits completely reversible amperometric response tosub-µM levels of CysNO, GSNO and other nitrosothiols.

### Example 24 Porcine models

A steel stent was uniformly coated using the photoreactive CVD polymer PPX-COPh. The stents were dipped into a solution containing cyclen methacrylate (30 wt%), HEMA (66%) and dimethacrylate (2%). overall 10% in methanol. They were then dried and exposed to UV radiation for 30 min. The stents were then incubated in CuCl2 solution (1mM in water) for 3 hours at 37 °C. The stents were washed repeatedly in DI-water. As a control stents were coated with the CVD polymer without the cyclone and with no copper exposure. The stents were then implanted in a live pig. 2 hour acute platelet deposition studies are shown in Figures 14A, B, C. Figure 14A shows a bare metal stent 2 hours after implantation, and indicates the formation of acute platelet deposition. Figure 14B shows a stent with a polymer coating only and also indicates the formation of acute platelet deposition. Figure 14C shows a stent of the invention with a CVD polymer covalently bound to a cyclen with copper showing minimal indication of acute platelet deposition.

### Example 25 FACS Analysis

After 3 days, the stents implanted in porcine models were analyzed using Fluorescent Activated Cell Sorting. A stent coated with a CVD polymer containing a nitric oxide generating agent was compared to a stent coated with a CVD polymer alone:

| | PCNA(Proliferating Cell Nuclear Antigen) | CD31 | Granulation |
|---|---|---|---|
| Nitric oxide generating agent +polymer stent | 29% | 20% | 81% |
| Polymer stent alone | 40% | 16% | 64% |

### References

### Patents and patent applications

US20030044546, US20020115559-A1
R. Virmani, F. D. Kolodgie, A. Farb, A. Lafont, Drug eluting stents: are human and animal studies comparable?, Heart 2003, 89 (2), 133-138; P. Libby, Inflammation in atheriosclerosis, Nature 2002, 420 (6917), 868-874; c) M.R. Bennett, M. O'Sullivan, Mechanisms of angioplasty and stent restenosis: implications for design of rational therapy, Pharmacology & Therapeutics, 2001, 91(2), 149-166; B. J. Rensing, W. R. Hermans, B. H. Strauss, P. W. Serruys, Regional differences in elastic recoil after percutaneous transluminal coronary angioplasty: a quantitative angiographic study, Journal of the American college of cardiology, 1991, 17, 34B-38B;C; Yutani, M. Imakita, H. Ishibashi-Ueda, Y. Tsukamoto, N. Nishida and Y. Ikeda, Coronary atherosclerosis and interventions: Pathological sequences and restenosis, Pathology International 1999; 49: 273-290; J. C. Palmaz, G. M. Richter, G. Noeldge, et al. Intraluminal stents in atherosclerotic iliacartery stenosis: preliminary report of a multicenter study. Radiology, 1988, 168,727-731; F. G. P. Welt, C. Rogers, Inflammation and restenosis in the stent era, Arteriosclerosis thrombosis and vascular biology 2002, 22(11), 1769-1776; S. J. Patel, D. Perera, S. R. Redwood, Stents and restenosis: the development of drug eluting stents, Journal of renovascular disease 2003, 2, 17-33; M. E. Collison, M. E. Meyerhoff, "Chemical Sensors for Bedside Monitoring of Critically III Patients." Anal. Chem. 1990, 62, 425A-437A;Espadas-Torre1 C.; Telting-Diaz, M.; Meyerhoff M.E. "Electrochemical Sensors for the Continuous Monitoring of Blood Gases." Interface 1994, Winter, 41-46; Turner, A.P.F.

## Claims

1. A medical device free of organic solvent capable of generating a pharmeceutically effective amount of nitric oxide and suitable for insertion into a mammal comprising:
a metal layer;
a coating with a thickness of 10 nm to 2000 nm on said metal layer; wherein said coating comprises a biocompatible polymer comprising at least one residue covalently bonded to a nitric oxide generating compound.

2. The medical device of claim 1, wherein said nitric oxide generating compound is a metal ion binding moiety.

3. The medical device of claim 1, wherein said nitric oxide generating compound is a Nₓ donor ligand where x is 2,3,4, 5, 6, 7, 8 or 9.

4. The medical device of claim 1, wherein said nitric oxide generating compound is a S_{y}-donor ligand where y is 2, 3, 4, 5, 6, 7, 8 or 9.

5. The medical device of claim 2 wherein said polymer comprises a (Nx-donor ligand-alkyl)-photoactive residue wherein said alkyl is a C₁₋₈ alkyl.

6. The medical device of claim 5, wherein said polymer comprises a (cyclen-alkyl)methylacrylate residue.

7. The medical device of claim 1, wherein said nitric oxide generating compound is dibenzo[e,k]-2,3,8,9-tetraphenyl-1,4,7,10-tetraaza-cyclododeca-1,3,7,9-tetraene; dibenzo[e,k]-2,3,8,9-tetramethyl-1,4,7,10 tetraaza-cyclododeca-1,3,7,9-tetraene; dibenzo[e,k]-2,3,8,9-tetraethyl-1,4,7,10-tetraaza-cyclododeca-1,3,7,9-tetraene, and/or salts thereof.

8. The medical device of claim 2, further comprising metal ions.

9. The medical device of claim 7, further comprising metal ions.

10. The medical device of claim 9, wherein said metal ions are selected from the ions of one or more of Cu, Co, Zn, Ca, Mg, Pt, Sn, Se, or Mn.

11. The medical device of claim 10, wherein said metal ion is Cu(II).

12. The medical device of claim 1, wherein said polymer comprises polymerized [2.2]paracyclophanes.

13. The medical device of claim 1, wherein said metal layer comprises at least one of stainless steel, titanium, copper, gold, nickel, or alloys thereof.

14. The medical device of claim 1 where said medical device is a catheter, a stent, a graft, or a pacemaker lead.

15. The medical device of claim 1, wherein said coating is 20 nm to 200 nm thick

16. The medical device of claim 1, further comprising another pharmaceutically effective agent.

17. The medical device of any of claims 1 to 16, wherein said medical device prevents an inflammatory response as compared to the inflammatory response obtained by implanting a medical device with a coating that does not include a nitric oxide generating compound.

## Patentansprüche

1. Medizinische Vorrichtung, die von organischem Lösungsmittel frei und in der Lage ist, eine pharmazeutisch wirksame Menge Stickstoffoxid zu bilden und zur Insertion in einen Säuger geeignet ist, umfassend:
eine Metallschicht;
eine Beschichtung mit einer Dicke von 10 nm bis 2000 nm auf der Metallschicht;
wobei die Beschichtung ein biokompatibles Polymer umfasst, das mindestens einen Rest umfasst, der kovalent an eine Stickstoffoxid bildende Verbindung gebunden ist.

2. Medizinische Vorrichtung nach Anspruch 1, wobei die Stickstoffoxid bildende Verbindung ein Metallionen bindender Anteil ist.

3. Medizinische Vorrichtung nach Anspruch 1, wobei die Stickstoffoxid bildende Verbindung ein Nₓ-Donatorligand ist, wobei x 2, 3, 4, 5, 6, 7, 8 oder 9 beträgt.

4. Medizinische Vorrichtung nach Anspruch 1, wobei die Stickstoffoxid bildende Verbindung ein Sy-Donatorligand ist, wobei y 2, 3, 4, 5, 6, 7, 8 oder 9 beträgt.

5. Medizinische Vorrichtung nach Anspruch 2, wobei das Polymer einen photoaktiven (Nₓ-Donatorligand-Alkyl-) Rest umfasst, wobei das Alkyl ein C₁₋₈-Alkyl ist.

6. Medizinische Vorrichtung nach Anspruch 5, wobei das Polymer einen (Cyclenalkyl)methylacrylatrest umfasst.

7. Medizinische Vorrichtung nach Anspruch 1, wobei die Stickstoffoxid bildende Verbindung Dibenzo[e,k]-2,3,8,9-tetraphenyl-1,4,7,10-tetraazacyclododeca-1,3,7,9-tetraen; Dibenzo[e,k]-2,3,8,9-tetramethyl-1,4,7,10-tetraazacyclododeca-1,3,7,9-tetraen; Dibenzo[e,k]-2,3,8,9-tetraethyl-1,4,7,10-tetraazacyclododeca-1,3,7,9-tetraen und/oder Salze davon ist.

8. Medizinische Vorrichtung nach Anspruch 2, des Weiteren Metallionen umfassend.

9. Medizinische Vorrichtung nach Anspruch 7, des Weiteren Metallionen umfassend.

10. Medizinische Vorrichtung nach Anspruch 9, wobei die Metallionen unter den Ionen eines oder mehrerer von: Cu, Co, Zn, Ca, Mg, Pt, Sn, Se oder Mn ausgewählt sind.

11. Medizinische Vorrichtung nach Anspruch 10, wobei das Metallion Cu(II) ist.

12. Medizinische Vorrichtung nach Anspruch 1, wobei das Polymer polymerisierte [2.2]Paracyclophane umfasst.

13. Medizinische Vorrichtung nach Anspruch 1, wobei die Metallschicht mindestens eines von Edelstahl, Titan, Kupfer, Gold, Nickel oder Legierungen davon umfasst.

14. Medizinische Vorrichtung nach Anspruch 1, wobei die medizinische Vorrichtung ein Katheter, ein Stent, ein Transplantat oder eine Schrittmacherleitung ist.

15. Medizinische Vorrichtung nach Anspruch 1, wobei die Beschichtung 20 nm bis 200 nm dick ist.

16. Medizinische Vorrichtung nach Anspruch 1, des Weiteren ein anderes pharmazeutisch wirksames Mittel umfassend.

17. Medizinische Vorrichtung nach einem der Ansprüche 1 bis 16, wobei die medizinische Vorrichtung eine Entzündungsreaktion verhindert im Vergleich mit der Entzündungsreaktion, die durch Implantieren einer medizinischen Vorrichtung mit einer Beschichtung, die keine Stickstoffoxid bildende Verbindung umfasst, erhalten wird.

## Revendications

1. Dispositif médical exempt de solvant organique capable de générer une quantité pharmaceutiquement efficace d'oxyde nitrique et approprié à l'insertion dans un mammifère comprenant:
une couche métallique;
un revêtement d'une épaisseur de 10 nm jusqu'à 2 000 nm sur ladite couche métallique; dans lequel ledit revêtement comprend un polymère biocompatible comprenant au moins un résidu lié de manière covalente à un composé générant de l'oxyde nitrique.

2. Dispositif médical selon la revendication 1, dans lequel ledit composé générant de l'oxyde nitrique est une fraction de liaison aux ions métalliques.

3. Dispositif médical selon la revendication 1, dans lequel ledit composé générant de l'oxyde nitrique est un ligand donneur de Nₓ où x prend la valeur de 2, 3, 4, 5, 6, 7, 8 ou 9.

4. Dispositif médical selon la revendication 1, dans lequel ledit composé générant de l'oxyde nitrique est un ligand donneur de S_{y} où y prend la valeur de 2, 3, 4, 5, 6, 7, 8 ou 9.

5. Dispositif médical selon la revendication 2, dans lequel ledit polymère comprend un résidu photoactif (donneur Nₓ ligand-alkyle) dans lequel ledit groupe alkyle est un groupe alkyle en C₁₋₈.

6. Dispositif médical selon la revendication 5, dans lequel ledit polymère comprend un résidu (cyclèn-alkyl)méthylacrylate.

7. Dispositif médical selon la revendication 1, dans lequel ledit composé générant de l'oxyde nitrique est le dibenzo[e,k]-2,3,8,9-tétraphényl-1,4,7,10-tétraaza-cyclododéca-1,3,7,9-tétraène; dibenzo[e,k]-2,3,8,9-tétraméthyl-1,4,7,10-tétraaza-cyclododéca-1,3,7,9-tétraène; dibenzo[e,k]-2,3,8,9-tétraéthyl-1,4,7,10-tétraaza-cyclododéca-1,3,7,9-tétraène, et/ou des sels de ceux-ci.

8. Dispositif médical selon la revendication 2, comprenant en outre des ions métalliques.

9. Dispositif médical selon la revendication 7, comprenant en outre des ions métalliques.

10. Dispositif médical selon la revendication 9, dans lequel lesdits ions métalliques sont choisis parmi les ions d'un ou plusieurs des éléments choisis parmi: Cu, Co, Zn, Ca, Mg, Pt, Sn, Se, ou Mn.

11. Dispositif médical selon la revendication 10, dans lequel ledit ion métallique est Cu(II).

12. Dispositif médical selon la revendication 1, dans lequel ledit polymère comprend des [2.2]paracyclophanes polymérisés.

13. Dispositif médical selon la revendication 1, dans lequel ladite couche métallique comprend au moins un parmi l'acier inoxydable, le titane, le cuivre, l'or, le nickel, ou les alliages de ceux-ci.

14. Dispositif médical selon la revendication 1, où ledit dispositif médical est un cathéter, une endoprothèse, une greffe, ou un conduit de pacemaker.

15. Dispositif médical selon la revendication 1, dans lequel ledit revêtement a une épaisseur de 20 nm à 200 nm.

16. Dispositif médical selon la revendication 1, comprenant en outre un autre agent pharmaceutiquement efficace.

17. Dispositif médical selon l'une quelconque des revendications 1 à 16, dans lequel ledit dispositif médical prévient une réponse inflammatoire telle que comparée à la réponse inflammatoire obtenue par l'implantation d'un dispositif médical avec un revêtement qui ne comprend pas de composé générant de l'oxyde nitrique.
